(19) Europäisches Patentamt
European Patent Office
Office européen des brevets

(11) Veröffentlichungsnummer: **0 325 941**
**A2**

(12) # EUROPÄISCHE PATENTANMELDUNG

(21) Anmeldenummer: **89100371.7**

(22) Anmeldetag: **11.01.89**

(51) Int. Cl.⁴: **C07D 229/00 , C08G 18/80**

(30) Priorität: **23.01.88 DE 3801934**

(43) Veröffentlichungstag der Anmeldung:
**02.08.89 Patentblatt 89/31**

(84) Benannte Vertragsstaaten:
**BE DE FR GB IT NL**

(71) Anmelder: **BAYER AG**
**D-5090 Leverkusen 1 Bayerwerk(DE)**

(72) Erfinder: **Scholl, Hans Joachim, Dr.**
**Am Feldrain 5**
**D-5000 Köln 80(DE)**
Erfinder: **Pedain, Josef, Dr.**
**Haferkamp 6**
**D-5000 Köln 80(DE)**
Erfinder: **Schönfelder, Manfred, Dr.**
**Höhenstrasse 126**
**D-5090 Leverkusen 3(DE)**

(54) **Verfahren zur Herstellung von modifizierten Polyisocyanaten, die nach diesem Verfahren erhältlichen Polyisocynate und ihre Verwendung.**

(57) Ein neues Verfahren zur Herstellung von Biuret- und Uretdiongruppen aufweisenden Polyisocyanaten durch Umsetzung von überschüssigen Mengen an (cyclo)aliphatischen Diisocyanaten mit einem Modifizierungsmittel bei 100 bis 210° C und anschließender Entfernung von nicht umgesetztem Ausgangsdiisocyanat, wobei man als Modifizierungsmittel 1,5-Diaminohexanol-6 oder dessen Lösungen in bis zu 80 Mol-%, bezogen auf Lösung, Wasser verwendet, die nach diesem Verfahren erhältlichen modifizierten Polyisocyanate und ihre Verwendung als Isocyanat-Komponente bei der Herstellung von Polyurethankunststoffen nach dem Isocyanat-Polyadditionsverfahren, insbesondere als Isocyanat-Komponente in Zweikomponenten-Polyurethanlacken.

EP 0 325 941 A2

# Verfahren zur Herstellung von modifizierten Polyisocyanaten, die nach diesem Verfahren erhältlichen Polyisocyanate und ihrer Verwendung

Die Erfindung betrifft ein neues Verfahren zur Herstellung von Biuret- und Uretdiongruppen aufweisenden organischen Polyisocyanaten durch Umsetzung von überschüssigen Mengen an (cyclo)aliphatischen Diisocyanaten mit einem speziellen Aminoalkohol oder mit dessen Lösungen in Wasser und anschließende Entfernung des nicht umgesetzten Ausgangsdiisocyanats, sowie die nach diesem Verfahren erhältlichen Verbindungen als auch ihre Verwendung zur Herstellung von Polyurethankunststoffen, insbesondere als Isocyanat-Komponente in Zweikomponenten-Polyurethanlacken.

Die Herstellung von modifizierten Polyisocyanaten durch Umsetzung von überschüssigen Mengen an (cyclo)aliphatischen Diisocyanaten, insbesondere an 1,6-Diisocyanatohexan mit Aminoalkoholen ist aus der DE-OS 2 641 448 bereits bekannt. Bei den Verfahrensprodukten dieser Vorveröffentlichung handelt es sich insbesondere um Biuret- und Urethan-modifizierte Polyisocyanate. Zwar wird in der DE-OS 2 641 448 auch darauf hingewiesen, daß die offenbarten Verbindungen Nebenprodukte, insbesondere Trimerisierungsprodukte (Isocyanurate), enthalten können, jedoch wird hierauf nicht weiter eingegangen. Die Anwesenheit von Uretdiongruppen in den Verfahrensprodukten der Vorveröffentlichung wird überhaupt nicht angesprochen. Die gemäß der Lehre der DE-OS 2 641 448 unter Verwendung von 1,6-Diisocyanatohexan und der als bevorzugt herausgestellten Aminoalkohole hergestellten, modifizierten Polyisocyanate weisen im übrigen, wie den Ausführungsbeispielen zu entnehmen, Viskositäten von mindestens 2400 mPa.s/25° C auf. Im allgemeinen liegt die Viskosität dieser Verfahrensprodukte jedoch noch weit höher und kann Werte von bis zu 43 000 mPa.s/25° C erreichen.

Es war nun die der Erfindung zugrundeliegende Aufgabe, ein neues Verfahren zur Herstellung von neuen, modifizierten aliphatischen Polyisocyanaten zur Verfügung zu stellen, wobei die neuen Polyisocyanate stabile, vergleichsweise niedrigviskose Flüssigkeiten darstellen sollten, die neben freien Isocyanatgruppen einen beträchtlichen Gehalt an dimerisierten Isocyanatgruppen (Uretdiongruppen) aufweisen sollten, die bei der Verwendung der Polyisocyanate als Harter in Zweikomponenten-Polyurethanlacken als potentielle, hitzeaktivierbare Isocyanatgruppen zur Verfügung stehen, wobei die Gesamtmenge an Isocyanatgruppen deutlich über dem Isocyanatgruppen-Gehalt der genannten Verfahrensprodukte des Standes der Technik liegt.

Diese Aufgabe konnte durch die Bereitstellung des nachstehend näher beschriebenen erfindungsgemäßen Verfahrens gelöst werden.

Gegenstand der Erfindung ist ein Verfahren zur Herstellung von Biuret- und Uretdiongruppen aufweisenden Polyisocyanaten durch Umsetzung von überschüssigen Mengen an (cyclo)aliphatischen Diisocyanaten mit einem Modifizierungsmittel bei 100 bis 210° C und anschließende Entfernung von nicht umgesetztem Ausgangsdiisocyanat, dadurch gekennzeichnet, daß man

(i) als Modifizierungsmittel 1,5-Diaminoheptanol-6 oder dessen Lösungen in bis zu 80 Mol-%, bezogen auf Lösung, Wasser und

(ii) der Ausgangsdiisocyanat in einem mindestens 15-fachen molaren Überschuß, bezogen auf (i), verwendet.

Gegenstand der Erfindung sind auch die nach diesem Verfahren erhältlichen Biuret- und Uretdiongruppen aufweisenden Polyisocyanate.

Gegenstand der Erfindung ist schließlich auch die Verwendung der Biuret- und Uretdiongruppen aufweisenden Polyisocyanate als Isocyanat-Komponente bei der Herstellung von Polyurethankunststoffen nach dem Isocyanat-Polyadditionsverfahren, insbesondere als Isocyanat-Komponente in Zweikomponenten-Polyurethanlacken.

Für das erfindungsgemäße Verfahren geeignete Ausgangsdiisocyanate sind beliebige organische Diisocyanate mit aliphatisch und/oder cycloaliphatisch gebundenen Isocyanatgruppen des Molekulargewichtsbereichs 140 bis 300 oder deren Gemische. Beispiele sind 1,4-Diisocyanatobutan, 1,6-Diisocycanatohexan, 1,6-Diisocyanato-2,2,4-trimethyl-hexan, 1,12-Diisocyanatododecan, Lysin-$C_1$-$C_8$-alkylester-diisocyanat, 1,3-Diisocyanatocyclobutan, 1,3-und/oder 1,4-Diisocyanatocyclohexan, 3,3′-Dimethyl-4,4′-diisocyanatodicyclohexylmethan, 4,4′-Diisocyanatodicyclohexylmethan, 1-Isocyanato-3,3,5-trimethyl-5-isocyanatomethylcyclohexan oder 1,4-Xylylendiisocyanat. Besonders bevorzugt wird 1,6-Diisocyanatohexan (HDI) als Ausgangsdiisocyanat verwendet.

Bei dem erfindungswesentlichen Aminoalkohol handelt es sich um 1,5-Diaminoheptanol-6 der Formel

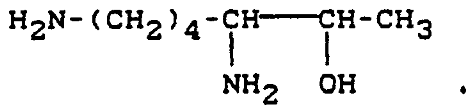

Die Herstellung dieser Verbindung kann beispielsweise dergestalt erfolgen, daß man L-Lysin in frieier Form und/oder in seiner HCl-Salz-Form mit Essigsäureanhydrid in Gegenwart einer tertiären organischen Aminbase und unter Zusatz eines 4-Aminopyridin-Derivates gemäß DE-A-3 425 814 zum 1,5-Disacetaminoheptanon-6 umsetzt. Das so erhältliche Produkt wird zum 1,5-Bisacetaminoheptanol-6 hydriert, nachfolgend durch Hydrolyse in wäßriger Mineralsäure in das Salz des 1,5-Diaminoheptanol-6 überführt und durch Behandlung mit einer starken Base als freies Amin 1,5-Diaminoheptanol-6 isoliert.

Eine beispielhafte Ausführungsform wird anhand des folgenden Formelschemas erläutert:

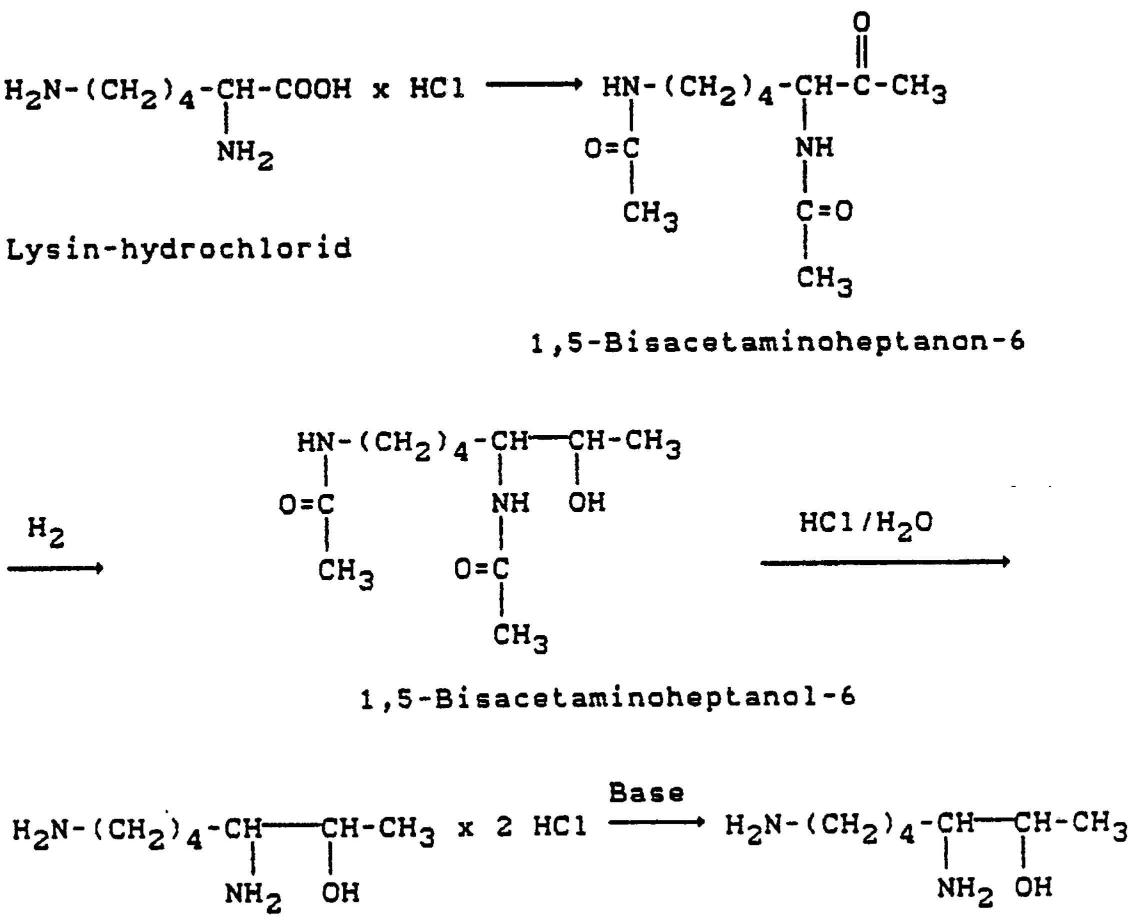


Lysin-hydrochlorid

1,5-Bisacetaminoheptanon-6

1,5-Bisacetaminoheptanol-6

1,5-Diaminoheptanol-6-hydrochlorid

1,5-Diaminoheptanol-6

Das in der ersten Stufe nach DE-A 3 425 814 erhältliche 1,5-Bisacetaminoheptanon-6 wird in der nächsten Stufe der an sich bekannten Hydrierung der Ketogruppe zur Hydroxylgruppe unterworfen; beispielsweise in Wasser als Lösungsmittel unter Verwendung von Raney-Nickel als Hydrierkatalysator bei Temperaturen von etwa 50 bis 150° C und einem Wasserstoffdruck von etwa 20 bis 50 bar.

Die so erhältliche wäßrige Lösung von 1,5-Bisacetaminoheptanol-6 wird, vorzugsweise nach Abtrennung des Katalysators, direkt der Hydrolyse in saurer Lösung (insbesondere in Mineralsäuren) zugeführt. Das 1,5-Bisacetaminoheptanol-6, ein Feststoff (Fp.: 105 bis 107° C) kann jedoch auch isoliert werden.

Die folgende Stufe besteht in der an sich bekannten Hydrolyse (Entacetylierung, vgl. DE-A 3 425 814) der N-Acetylgruppen in wäßriger, mineralsauer Lösung zu den entsprechenden, primäre Aminogruppen enthaltenden Salzen des 1,5-Diaminoheptanol-6, beispielsweise in 4- bis 6-normaler, wäßriger Salzsäurelösung durch 4- bis 6-stündiges Erhitzen unter Rückflußbedingungen. Nachfolgendes Einengen und Umkristallisieren führt zu gereinigten Salzen des 1,5-Diaminoheptanol-6. Ihre Umsetzung mit NaOH oder KOH aus konzentrierten wäßrigen Lösungen führt zum reinen, freien Amin, das jedoch auch unter Umgehung der Aminsalz-Isolierung direkt mit NaOH oder KOH als Rohamin abgeschieden werden kann. Eine weitere Reinigung ist durch Destillation möglich.

Beim erfindungsgemäßen Verfahren wird 1,5-Diaminoheptanol-6 in Substanz oder in Form einer wäßrigen Lösung als Modifizierungsmittel für das Ausgangsdiisocyanat verwendet. So können beispielsweise wäßrige Lösungen des Aminoalkohols mit einem Gehalt von bis zu 80 Mol-%, vorzugsweise bis zu 70 Mol-% Wasser, bezogen auf die Lösung, als Modifizierungsmittel verwendet werden.

Das erfindungsgemäße Verfahren wird im Temperaturbereich von 100 bis 210° C, vorzugsweise 100 bis 190° C durchgeführt. Bei der Durchführung des erfindungsgemäßen Verfahrens gelangen pro Mol der in der Komponente (i) vorliegenden Verbindungen (Summe der Mole Aminoalkohol + der Mole Wasser) mindestens 15 Mol, vorzugsweise 30 bis 60 Mol Diisocyanat zum Einsatz.

Die Durchführung des erfindungsgemäßen Verfahrens kann beispielsweise folgendermaßen erfolgen:

Das als Ausgangsmaterial eingesetzte, aliphatische oder cycloaliphatische Diisocyanat wird in einem Rührgefäß unter Inertgas-Atmosphäre (z.B. Stickstoff oder Argon) vorgelegt und auf 120 bis 190° C erwärmt. 1,5-Diamino-6-heptanol wird, gegebenenfalls in Form einer wäßrigen Lösung, hinzugefügt und das so erhaltene Reaktionsgemisch so lange bei 120° C bis 190° C gerührt, bis die Reaktionslösung homogen ist und der gewünschte Brechungsindex (der gegebenenfalls in Vorversuchen für die jeweilige Reaktionsmischung und dem jeweils angestrebten Gehalt an Uretdiongruppen festzulegen ist) erreicht ist. Auf diese Weise erhält man Biuret-, Urethan- und Isocyanuratgruppen aufweisende Polyisocyanate mit einem gezielt einstellbaren Gehalt an Uretdiongruppen, wie mittels der $^{13}C$-Spektroskopie nachweisbar ist (vgl. Angew. Makromol. Chem. 141 (1986) 173-183). Das erfindungsgemäße Verfahren wird vorzugsweise lösungsmittelfrei durchgeführt.

Nach Beendigung der Reaktion wird das überschüssige Ausgangsdiisocyanat durch Extraktion, beispielsweise unter Verwendung von n-Hexan als Extraktionsmittel, oder vorzugsweise durch Destillation (Dünnschichtverdampfer) bis auf einen Restgehalt von max. 1, vorzugsweise max. 0,5 Gew.-%, entfernt.

Die erfindungsgemäßen Verfahrensprodukte stellen farblose bis gelblich gefärbte, bei Raumtemperatur flüssige Polyisocyanate dar. Sie sind völlig geruchlos und klar in gegenüber Isocyanatgruppen inerten Lösungsmitteln wie Kohlenwasserstoffen, Chlorkohlenwasserstoffen, Estern oder Ketonen löslich.

Die bevorzugten erfindungsgemäßen Verfahrensprodukte auf Basis von 1,6-Diisocyanatohexan weisen bei 23° C eine Viskosität von 300 bis 5000 mPa.s, einen NCO-Gehalt von 20 bis 24 Gew.-% und einen Gehalt an Uretdiongruppen (berechnet als $C_2N_2O_2$) von 4 bis 20 Gew.-% auf. Neben diesen Gruppierungen liegen in den erfindungsgemäßen Verfahrensprodukten Biuret-, Urethan- und Isocyanatgruppen sowie gegebenenfalls Allophanatgruppen vor.

Die erfindungsgemäßen Verfahrensprodukte unterscheiden sich von den Produkten der DE-OS 2 641 448 vorteilhaft durch eine niedrigere Viskosität und gleichzeitig (insbesondere unter Berücksichtigung der neben den freien Isocyanatgruppen vorliegenden Uretdiongruppen) durch eine höhere NCO-Funktionalität und einen höheren NCO-Gehalt.

Die erfindungsgemäßen Verfahrensprodukte stellen wertvolle Ausgangsmaterialien zur Herstellung von Polyurethankunststoffen dar. Insbesondere eignen sie sich zur Herstellung von hochwertigen, lichtechten Zweikomponenten-Polyurethanlacken. In mit Blockierungsmitteln für Isocyanatgruppen blockierter Form eignen sie sich auch zur Herstellung von Polyurethan-Einbrennlacken.

In den nachfolgenden Beispielen beziehen sich alle Prozentangaben, soweit nichts anderslautendes vermerkt, auf Gewichtsprozente.

Beispiel 1 (Herstellung von 1,5-Dimaninoheptanol-6)

a) 1,5-Bisacetaminoheptanon-6

In eine Mischung aus 3676 g Essigsäureanhydrid, 2429 g Triethylamin und 2,4 g N,N-Dimethyl-4-amino-pyridin werden bei 40 bis 50° C portionsweise 1096 g L-Lysin-hydrochlorid unter Rühren eingetragen. Nachdem ca. 80 l $CO_2$ entwichen sind, wird solange bei 60 bis 70° C nachgerührt, bis insgesamt ca. 135 l $CO_2$ abgespalten wurden. Man filtriert von ausgefallenen Triethylaminohydrochlorid ab, befreit das Filtrat im Vakuum von niedrigsiedenden Anteilen und kristallisiert das verbleibende Rohprodukt aus Essigester. Man erhält 1163 g 1,5-Bisacetaminoheptanon-6 vom Fp. 108-110° C.

b) 1,5-Bisacetaminoheptanol-6

In einem Rührautoklav werden 456 g 1,5-Bisacetaminoheptanon-6 in 1,5 l Wasser gelöst und mit 50 g Raney-Nickel versetzt. Bei 90° C und 40 bis 60 bar Wasserstoffdruck wird bis zum Ende der Wasserstoffaufnahme gerührt. Anschließend wird entspannt, vom Katalysator abfiltriert und die wäßrige Lösung von 1,5-Bisacetaminoheptanol-6 direkt der sauren Hydrolyse zugeführt.

c) 1,5-Diaminoheptanol-6

Die wäßrige Lösung aus b) wird mit 1,5 kg 37 %iger Salzsäure versetzt, 6 Stunden unter Rückflußbedingungen gehalten und nachfolgend im Vakuum von niedrigsiedenden Anteilen befreit. Das erhaltene Rohprodukt 1,5-Diaminoheptanol-6-bishydrochlorid wird nachfolgend unter Kühlung mit 480 g 50 %iger Natronlauge versetzt. Man trennt vom entstandenen Natriumchlorid und überschüssiger Natronlauge ab und reinigt das so isolierte Rohamin, das lediglich nichtdestillierbare Verunreinigungen und geringe Anteile an Wasser enthält, durch Destillation. Auf diese Weise können 251 g 1,5-Diaminoheptanol-6 als bei 113 bis 115° C/0,1 mbar siedende Substanz erhalten werden (Ausbeute 86 %).

| Analyse (%): | | | |
|---|---|---|---|
| | C | H | N |
| gefunden | 57,4 | 12,5 | 19,1 |
| Theorie | 57,5 | 12,3 | 19,2 |
| (bezogen auf $C_7H_{18}N_2O$) | | | |

Beispiel 2 (erfindungsgemäßes Verfahren)

1680 g (10 Mol) HDI werden unter Stickstoffatmosphäre auf 180° C erhitzt. Anschließend läßt man 29,2 g (0,2 Mol) 1,5-Diaminoheptanol-6 unter Rühren bei 180° C während 1 h zutropfen und 10 Minuten bei 180° C nachrühren. Die klare Lösung wird anschließend bei 120° C so lange gerührt (2 bis 6 h), bis der Brechungsindex $n_D^{25}$ = 1,4600 erreicht ist. Anschließend trennt man das überschüssige HDI durch Dünnschichtdestillation (Typ "Kurzwegverdampfer") bei 120° C/0,1 mbar bis auf einen Restgehalt von 0,1 % ab.

Ausbeute: 370 g
Viskosität: 1000 mPa.s/23° C
NCO-Gehalt: 23,5 %
Uretdion-Gehalt: 13 %
Zusammensetzung nach $^{13}C$-NMR (Mol-%):
Biuretgruppen: 51 %; Uretdiongruppen: 33 %;
Isocyanuratgruppen: 16 %.

Diese Angaben in "Mol-%" beziehen sich hier und auch in den nachfolgenden Beispielen auf den Gesamtgehalt der Verfahrensprodukte an den genannten Gruppen.

Beispiel 3

2520 g (15 Mol) HDI werden unter Stickstoffatmosphäre auf 160° C erhitzt und innerhalb von 1 h mit 43,8 g (0,3 Mol) 1,5-Diaminoheptanol-6 unter Rühren versetzt. Man rührt 1 h bei 160° C nach, kühlt auf 120° C und rührt so lange bei dieser Temperatur, bis der Brechungsindex $n_D^{25}$ = 1,4610 erreicht ist (2 bis 3 h). Nach Aufarbeitung gemäß Beispiel 2 erhält man ein Produkt mit folgenden Daten:

Ausbeute: 551 g
Viskosität: 1700 mPa.s/23° C
NCO-Gehalt: 23,7 %
Uretdion-Gehalt: 12 %

Zusammensetzung nach $^{13}C$-NMR (Mol-%):
Biuretgruppen: 48,8 %; Uretdiongruppen: 30,5 %;
Isocyanuratgruppen: 20,7 %.

Beispiel 4

Gemäß Beispiel 2 werden 1512 g (9 Mol) HDI und 43,8 g (0,3 Mol) 1,5-Diaminoheptanol-6 zur Umsetzung gebracht. Nach Erreichen des Brechungsindex: $n_D^{25}$ = 1,4750 wird das Produkt entsprechend aufgearbeitet.
Ausbeute: 675 g
Viskosität: 6200 mPa.s/23° C
NCO-Gehalt: 21,9 %
Uretdion-Gehalt: 5 %
Zusammensetzung nach $^{13}C$-NMR (Mol-%):
Biuretgruppen: 31,0 %; Uretdiongruppen: 14,2 %;
Isocyanuratgruppen: 54,8 %.

Beispiel 5

Gemäß Beispiel 2 werden 1680 g (10 Mol) HDI und eine Lösung aus 21,9 g (0,15 Mol) 1,5-Diaminoheptanol-6/2,7 g (0,15 Mol) Wasser zur Umsetzung gebracht. Man rührt 1 h bei 180° C nach und beläßt die klare Lösung anschließend solange bei 120° C (1 bis 3 h) bis der Brechungsindex: $n_D^{25}$ = 1,4620 erreicht ist. Nach Aufarbeitung erhält man ein Produkt mit den Daten:
Ausbeute: 372 g
Viskosität: 1200 mPa.s/23° C
NCO-Gehalt: 23,8 %
Uretdion-Gehalt: 11 %
Zusammensetzung nach $^{13}C$-NMR (Mol-%):
Biuretgruppen: 48,5 %; Uretdiongruppen: 27 %;
Isocyanuratgruppen: 24,5 %.

Beispiel 6

Gemäß Beispiel 2 werden 1680 g (10 Mol) HDI und eine Lösung aus 29,2 g (0,2 Mol) 1,5-Diaminoheptanol-6/1,8 g (0,1 Mol) Wasser zur Umsetzung gebracht. Nach Erreichen eines Brechungsindex: $n_D^{25}$ = 1,4615 wird gemäß Beispiel 2 aufgearbeitet.
Ausbeute: 375 g
Viskosität: 2700 mPa.s/23° C
NCO-Gehalt: 23,8 %
Uretdion-Gehalt: 8 %
Zusammensetzung nach $^{13}C$-NMR (Mol-%);
Biuretgruppen: 57,9 %, Uretdiongruppen: 18,3 %;
Isocyanuretgruppen: 23,8 %.

Beispiel 7

Gemäß Beispiel 6 werden 1680 g (10 Mol) HDI und eine Lösung aus 18,25 g (0,125 Mol) 1,5-Diaminoheptanol-6/2,25 g (0,125 Mol) Wasser zur Umsetzung gebracht. Nach Erreichen eines Brechungsindex: $n_D^{25}$ = 1,4588 wird gemäß Beispiel 6 aufgearbeitet.
Ausbeute: 299 g
Viskosität: 980 mPas/23° C
NCO-Gehalt: 24,3 %
Uretdion-Gehalt: 10 %
Zusammensetzung nach $^{13}C$-NMR (Mol-%):

Biuretgruppen: 66,1 %; Uretdiongruppen 21,2 %;
Isocyanuretgruppen 12,7 %.

Beispiel 8 (erfindungsgemäße Verwendung)

Das Polyisocyanat aus Beispiel 7 wird zu einem 2-Komponenten-PUR-Lack verarbeitet, indem es mit einer Hydroxylkomponente A, die das Gemisch eines Polyesters mit einem Hydroxypolyacrylat darstellt, kombiniert wird.

Zur Herstellung der Hydroxylkomponente A wird wie folgt vorgegangen:

260 Gewichtsteile eines Polyesters aus 2,5 Mol Trimethylolpropan, 1,65 Mol Phthalsäureanhydrid, 0,6 Mol Adipinsäure und 1,15 Mol 2-Ethylhexansäure werden in Lösung mit 390 Gewichtsteilen eines Polymerisats aus 39,63 Mol-% Styrol, 28,01 Mol-% n-Butylacrylat, 0,94 Mol-% Acrylsäure und 31,42 Mol-% Hydroxyethylmethacrylat abgemischt. Das Gemisch ist 65 %ig in Xylol/Butylacetat (Gewichtsverhältnis (1:3) gelöst. Die Lösung hat eine Hydroxylzahl von 95.

Das Polyisocyanat wird mit der Hydroxylkomponente A unpigmentiert und pigmentiert (mit Titandioxid vom Rutil-Typ, das über einen Dreiwalzenmischer eingearbeitet wird und im fertigen Lack in einer Menge von ca. 80 Gew.-Teilen pro 100 Gew.-Teilen an Polyurethan-bildenden Ausgangsmaterialien, enthalten ist) im NCO/OH-Verhältnis 1:1 gemischt. Mit Methoxypropylacetat wird auf eine Spritzviskosität von 18 Sekunden eingestellt (nach DIN 53 211, 4 mm). Die spritzfertige Lacklösung wird auf Flußstahlplatten gespritzt und unter verschiedenen Bedingungen ausgehärtet (vgl. nachfolgende Tabelle).

Tabelle

| | unpigmentiert | pigmentiert |
|---|---|---|
| Feststoffgehalt bei Spritzviskosität 18 | 54 % | 62 % |
| Verarbeitungszeit | 3,7 h | 10,6 h |
| Pendelhärte (s) (nach DIN 53 157) nach Aushärtung bei | | |
| 30 min/80 °C<br>30 min/120 °C<br>30 min/80 °C + 16 h/60 °C<br>7 Tage bei ca. 23 °C | 179<br>210<br>210<br>204 | 185<br>194<br>198<br>187 |
| Lösemittelbeständigkeit* nach | | |
| 30 min/120 °C und Alterung 7 Tage bei ca. 23 °C | 0,0,0,1 | 0,0,0,1 |

Erläuterungen der Tabelle:

* Lösemittelbeständigkeit

Die Anlösbarkeit der Lackfilme wird beurteilt nach 1 min Einwirkzeit der Lösemittel. Die Schädigung des Lackfilms wird in 6 Stunden beurteilt von

0 = Lackfilm ist völlig unverändert bis

5 = Lackfilm löst sich auf.

In der angegebenen Reihenfolge kommen folgende Lösemittel zur Anwendung:

Toluol
Methoxypropylacetat
Ethylacetat
Aceton

## Ansprüche

1. Verfahren zur Herstellung von Biuret- und Uretdiongruppen aufweisenden Polyisocyanaten durch Umsetzung von überschüssigen Mengen an (cyclo)aliphatischen Diisocyanaten mit einem Modifizierungsmittel bei 100 bis 210° C und anschließende Entfernung von nicht umgesetztem Ausgangsdiisocyanat, dadurch gekennzeichnet, daß man
(i) als Modifizierungsmittel 1,5-Diaminoheptanol-6 oder dessen Lösungen in bis zu 80 Mol-%, bezogen auf Lösung, Wasser und
(ii) das Ausgangsdiisocyanat in einem mindestens 15-fachen molaren Überschuß, bezogen auf (i), verwendet.

2. Verfahren gemäß Anspruch 1, dadurch gekennzeichnet, daß man als Ausgangsdiisocyanat 1,6-Diisocyanatohexan verwendet.

3. Verfahren gemäß Anspruch 2, dadurch gekennzeichnet, daß man das 1,6-Diisocyanatohexan in einem 30- bis 60-fachen molaren Überschuß, bezogen auf (i) verwendet.

4. Gemäß Anspruch 1 bis 3 erhältliche, modifizierte Polyisocyanate.

5. Verwendung der gemäß Anspruch 1 bis 3 erhältlichen, modifizierten Polyisocyanate als Isocyanat-Komponente bei der Herstellung von Polyurethankunststoffen nach dem Isocyanat-Polyadditionsverfahren.

6. Verwendung gemäß Anspruch 5 als Isocyanat-Komponente in Zweikomponenten-Polyurethanlacken.